(19) Europäisches Patentamt
European Patent Office
Office européen des brevets

(11) Publication number: **0 084 678**
**B1**

(12) # EUROPEAN PATENT SPECIFICATION

(45) Date of publication of patent specification: **24.04.85**

(51) Int. Cl.[4]: **C 07 C 69/90**, C 07 C 67/14, A 61 K 31/235

(21) Application number: **82112027.6**

(22) Date of filing: **27.12.82**

(54) **New compound having analgesic, antiinflammatory and mucoregulating activities, process for its production and relative pharmaceutical compositions.**

(30) Priority: **21.01.82 IT 1921582**

(43) Date of publication of application:
**03.08.83 Bulletin 83/31**

(45) Publication of the grant of the patent:
**24.04.85 Bulletin 85/17**

(84) Designated Contracting States:
**AT BE CH DE FR GB IT LI LU NL SE**

(56) References cited:
**None**

(73) Proprietor: **AUSONIA FARMACEUTICI S.r.l.**
**Via Laurentina Km. 24730**
**I-00040 Pomezia (Rome) (IT)**

(72) Inventor: **de Vincentiis, Leonardo**
**Via Isonzo 42**
**Roma (IT)**

(74) Representative: **Bianchetti, Giuseppe**
**Studio Consulenza Brevettuale Via Senato 24**
**I-20121 Milan (IT)**

**EP 0 084 678 B1**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

## Description

It is an object of the present invention 2-acetoxybenzoate of 2-methyl-5-(1-hydroxy-1-methylethyl)-cyclohexen-2-yl, having formula (I)

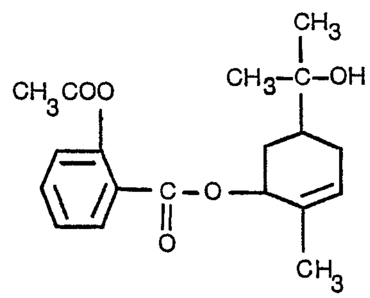

(I)

The therapeutic properties of 2-methyl-5-(1-hydroxy-1-methyl-ethyl)-2-cyclohexen-1-ol, or sobrerol (II)

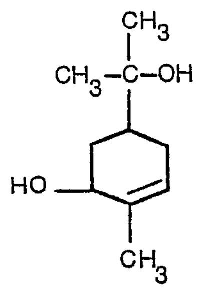

(II)

are known since many years. It has been used for the treatment of respiratory diseases (V. Dalla Valle — Boll. Chim. Farm., 109, (1970)), and more exactly as fluidifying agent with bronchosecretagogue and mucosecretolytic activity in cases of acute and chronic bronchitis with hypersecretive and obstructive component of the respiratory routes (R. Scuri et al., Il Farmaco ed Pr.*36*, 1 (1980)). On the other side, the pharmacological and therapeutic properties of 2-acetoxy-benzoic, or acetylsalicylic acid (ASA), are known to such an extent that is needless to dwell upon any longer.

It has now unexpectedly found that the ester of acetylsalicylic acid with sobrerol, that is compound (I), while exhibiting a bronchosecretogogue activity higher than that of sobrerol and a mucosecretolytic and fluidifying activity comparable to that sobrerol itself, is moreover characterized by analgesic and antiinflammatory properties, comparable to that of ASA, in comparison with which it has however the advantage of a definitely lower gastrolesivity.

Thus, another object of the invention is provided by pharmaceutical compositions endowed with antiinflammatory, analgesic and mucoregolating and anticough activities, containing as active principle compound (I).

Finally, the invention relates to a process for the preparation of 2-acetoxybenzoate of 2-methyl-5-(1-hydroxyl-1-methyl-ethyl)cyclohexen-2-yl, characterized by reacting 2-acetoxybenzoic acid chloride (III) with sobrerol (II) according to the following scheme:

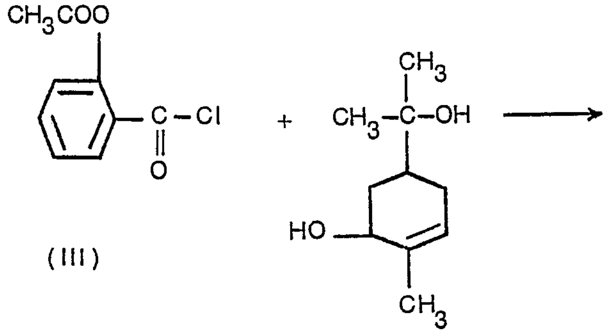

(III) (II) (I)

The reaction is suitably carried out in aprotic solvents, for example in ethers such as diethylether, tetrahydrofuran, dioxane, and in presence of acidity acceptors. As such, tertiary bases are preferably used, such as pyridine or triethylamine. One can operate at temperatures ranging from 10 to 100°C, preferably from about 20 and 80°C.

The following example further illustrates, without limiting it, the process according to the invention.

Example

To 90 g of ASA in 500 ml of benzene, 120 g of thionyl chloride are added. The mixture is refluxed for three hours. At the end, the benzene is evaporated under reduced pressure to dryness. The acetylsalicyclic acid chloride so obtained is dissolved in 500 ml of anhydrous tetrahydrofuran and added with 80 g of pyridine; to this solution 85 g of sobrerol (dl-trans form) are added. The reaction is completed after two hours refluxing. At the end the solvent is evaporated under reduced pressure, the residue is washed with water and extracted with ethyl ether. The organic phase is washed with 0,5 N HCl and thereafter with water, then it is dried on anhydrous sodium sulphate and freed from solvent under reduced pressure. The crude product (130 g) is purified by chromatography on Florisil (100—200 mesh) eluting with petroleum ether and then washed with petroleum ether/ethyl ether 2:1. 75 Grams of a dense, yellowish oil are obtained, slightly soluble in water and easily soluble in organic solvents, pure in TLC. Its structure is confirmed by analytical and spectroscopical data:

Elemental analysis: for $C_{19}H_{24}O_5$ (M.W. = 332.39)
calcd. % C = 68.66; H = 7.28
found % C = 68.79; H = 7.33.
I.R. Spectrum (liquid film; values of absorption bands are expressed in $cm^{-1}$):
stretch O—H 3550—3400 broad bands
stretch C—H aromatics alifatics 3080—2910
stretch C = O alifat. ester 1780
stretch C = O aromat. ester 1730
stretch C = C 1615
stretch C(= O)—O acetate 1145
stretch C(= O)—O arilyc 1255

$H^1$ NMR Spectrum (recorded in $CDCl_3$, internal references TMS; values of chemical shifts are expressed in δ):
1.2 (d, 6H, 2($CH_3$));
1.8 (s, 3H, =C—$CH_3$);
1.4—2.2 (m, 6H, $CH_2$—CH—$CH_2$, OH mobile);
2.3 (s, 3H, CO—$CH_3$);
5.3 (m, 1H, CH—O—CO);
5.8 (m, 1H, <u>CH</u>=C—$CH_3$);
7—7.7 (m, 3H aromatics);
8 (double d, 1H aromatic).

The bio-pharmaceutical characteristics of compound (I) which will be hereinafter defined, for the sake of brevity, with the abbreviation AFP 696 have been determined as hereinafter described.

Toxicity

The acute toxicity has been determined in Swiss mouse and in Wistar rat for different administration routes, according to the method of Litchfield J.T. and Wilcoxon [J. Pharm. Exp. Therap. *96*, 99—113, (1949)].

The results obtained are reported in the following Table I; from the same it can be pointed out how AFP 696 is endowed with a very low acute toxicity.

TABLE I — Acute toxicity of AFP 696

| Species | Administration route | $LD_{50}$ — (mg/kg) |
|---|---|---|
| mouse | os | 3500 |
| | i.p. | 1600 |
| rat | os | >4000 |
| | i.p. | 1950 |

Bronchosecretogogue activity

The bronchosecretogogue activity has been determined in male Wistar rats by per os administration according to the method of Mawatari [Mawatari H., Kagoshima Daigaku Igaku-Zasshi, *27*, 561 (1976)], comparing AFP-696 with carboxymethylcysteine and sobrerol at the doses hereinafter stated. The results are reported in the enclosed Table 2.

TABLE 2

Bronchosecretogogue activity (Mawatari technique)

| Compound | Administered Dose mg/kg/os | No of animals | % Increase of FlNa (*) in comparison with controls |
|---|---|---|---|
| AFP-696 | 500 | 10 | 63.6 |
| Carboxymethyl-cysteine | 500 | 10 | 39.7 |
| Sobrerol | 500 | 10 | 33.4 |

(*) FlNa = Sodium fluoresceine.

From the resulting values a more marked activity of AFP-696 in the test under exam clearly turns out in comparison with reference drugs used at equiponderant doses.

Mucosecretolytic activity

The mucosecretolytic activity has been studied in the male rabbit according to the technique of Giraldez, Grass and Bruseghini (Abstr. Comp. Int. Pharm. N. 1086-Paris 1978), by administration by the oral route, comparing AFP-696 with carboxymethylcysteine and sobrerol at the dose hereinafter stated. The results are reported in the Table 3.

TABLE 3

Mucosecretolytic activity (technique of Giraldez et al.)

| Compound | Dose mg/kg/os | No animals | % Increase of secretion in comparison with controls |
|---|---|---|---|
| AFP—696 | 500 | 10 | 39.5 |
| Carboxymethyl-cysteine | 500 | 10 | 40.2 |
| Sobrerol | 500 | 10 | 36.8 |

From the comparison of the results obtained by the test under exam the mucosecretolytic activity of AFP-696 results comparable to that of carboxymethylcysteine and sobrerol used at equiponderant doses.

Antiinflammatory activity

a) Carrageenin edema

Tests have been performed on the rat, employing the test of the subplantar edema induced by carrageenin. AFP-695 and, for comparison, ASA, have been administered by oral route 30 minutes before carrageenin, at the doses hereinafter stated. After edema induction, the animals of each group have been controlled, every 60 minutes for 5 consecutives hours, in order to point out the evolution of the inflammatory process.

The results obtained, expressed as volume of edematous limbs, are reported in the Table 4. From the comparison between the values of control rats and those of AFP-696 treated rats, it turns out the very good antiinflammatory activity of the latter, comparable to the that of ASA, in the used experimental conditions and at the used doses.

TABLE 4

Antiinflammatory activity — subplantar edema by carrageenin in the rat.

| Treatment | Dose mg/kg os | MEAN LIMB VOLUME AT DIFFERENT HOURS AFTER TREATMENT | | | | | | AREA | |
|---|---|---|---|---|---|---|---|---|---|
| | | 0 | 1 | 2 | 3 | 4 | 5 | Absolute value | % Inhibition in comparison with controls |
| Controls | — | 23.8 | 30.7 | 35.1 | 40.2 | 43.7 | 44.8 | 273.1 | — |
| Acetylsalicylic acid | 250 | 24.3 | 27.4 | 32.2 | 37.8 | 38.6 | 38.6 | 181.28 | 33.6 |
| AFP-696 | 500 | 24.1 | 28.1 | 31.4 | 37.4 | 38.1 | 38.1 | 183.0 | 33.0 |

Gastrolesive action

Rats fasting since 18 hours have been treated by oral route with AFP-696 and, for comparison, with acetylsalicylic acid, at the doses hereinafter stated. 5 Hours and 30 minutes after treatments, the animals have been sacrificed and the stomach has been withdrawn for gastric mucose examination.

As it is possible to evidence from the results obtained, reported in the Table 5, the gastrolesivity of AFP-696, in the used experimental conditions, is by far lower than that of the reference drugs.

TABLE 5

Gastrolesive action

| Treatment | Dose mg/kg/os | Mean ulcers' size (mm) |
|---|---|---|
| Controls | — | 0 |
| Acetylsalicylic acid | 100 | 3.9 ± 1.2 |
| AFP-696 | 150 | 0.7 ± 0.4 |

Analgesic activity

The test of writhings by phenylquinone in the mouse has been used. AFP-696 has been administered by oral route at the dose of 500 mg/kg in comparison with acetylsalicylic acid at the dose of 250 mg.

The two drugs have been administered per os 30 minutes after intraperitoneal injection of phenilquinone.

The very good analgesic activity of AFP-696, slightly lower than that of acetylsalicylic acid administered at equimolar doses, is shown from the results obtained and reported in the Table 6.

TABLE 6

Analgesic activity

| Treatment | Dose mg/kg os | Mean No of writhings | % Inhibition in comparison with controls | No animals with writhings |
|---|---|---|---|---|
| Controls | — | 12.8 ± 2.6 | — | 10/10 |
| Acetylsalicylic acid | 250 | 0.6 ± 0.5 | 95.3 | 1/10 |
| AFP-696 | 500 | 1.3 ± 0.9 | 89.8 | 3/10 |

Anti-cough activity

The anti-cough activity of AFP-696 has been assessed after administration by endoperitoneal route (30% in tween) on rabbits of both sexes, weighing 200—350 g, according to the method of the cough induced by citric acid aerosol administration, and evaluating the mean number of cough strokes in comparison with the same basal values (Boura and coll. — Prog. Brit. Pharmac. Soc. Apr. 1970). As reference standard a group of codeine treated animals at the dose of 12.5 mg/kg has been used.

TABLE 7

Anti-cough activity

| Treatment | Dose mg/kg | No animals | % Inhibition in comparison with controls | Statistical significance (basal minus final values) |
|---|---|---|---|---|
| Controls | — | 12 | 26.7 | — |
| Codeine | 12.5 | 12 | 76.7 | $p < 0.05$ |
| AFP-696 | 150 | 12 | 82.4 | $p < 0.05$ |

From the above stated results it becomes evident the very good anti-cough activity of AFP-696 in comparison with traditional standard of proved activity, in the used experimental conditions.

Pharmacokinetics

Two groups of rats have been treated per os, by gastric tube, with AFP-696 at the dose of 100 mg/kg and acetylsalicylic acid at the dose of 54 mg/kg. At the moment of treatment rats were fasting since 18 hours. Rats have been then sacrificed in groups of 4 at different times: 30 min., 1 h, 2 h, 4 h, 6 h.

The concentration both of unchanged products and of their metabolites have been measured by gaschromatography in plasma and in the lung. At 30 minutes, and in some instance also at 1 hour, unchanged AFP-696 has been found in plasma showing that this product is at least partially absorbed as such.

The plasma levels of acetylsalicylic acid proved to be higher after administration of AFP-696 in comparison with the administration of acetylsalicylic acid at equimolar doses. The difference was more marked in the first two hours and it is relative to a better bioavailability for acetylsalicylic acid of about 20%.

Also pulmonary levels of salicylic acid proved to be higher after administration of AFP-696.

Concentration ratios between lung and plasma in the case of salicylic acid were 0.8 for the administration of acetylsalicylic acid, and 1.1 for the administration of AFP-696.

The experiment shows a particularly good oral bioavailability and a clear tropism for the lung.

The compound (I) is therefore fit as an elective drug with analgesic, antiinflammatory, mucoregulating and anti-cough, in the therapy of acute and chronic catarrhal diseases of respiratory apparatus, also ensuing from influenza and cold conditions.

The present invention refers also to all the industrially applicable aspects connected with the use of AFP-696 as analgesic, antiinflammatory, mucoregulating and anti-cough agent. An essential aspect of the invention is therefore provided by pharmaceutical compositions containing predetermined amounts of AFP-696. Such a compound can be administered, particularly, by the oral route, as capsules, extemporary suspension, monodose sachets (in a granular state); or by aerosol; or as suppositories; or, finally, by parenteral route (vials of injection).

Of course, the single pharmaceutical forms will contain the active principle together with vehicles, excipients, flavouring agents etc. of conventional use in pharmaceutical technique.

**Claims for the Contracting States: BE CH DE FR GB IT LI LU NL SE**

1. As a novel compound, 2-acetoxybenzoate of 2-methyl-5-(1-hydroxy-1-methyl-ethyl)-cyclohexen-2-yl, having formula (I)

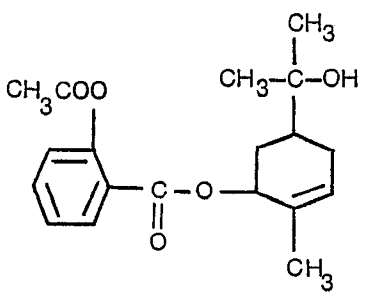

(I)

2. Pharmaceutical composition endowed with analgesic, antiinflammatory, mucoregulating and anti-cough activities, characterized by containing as active principle the compound of claim 1.

3. Process for the preparation of 2-acetoxybenzoate of 2-methyl-5-(1-hydroxy-1-methyl-ethyl)-cyclohexen-2-yl, having formula (I), characterized by reacting acetylsalicylic acid chloride (III) with sobrerol (II), according to the following scheme:

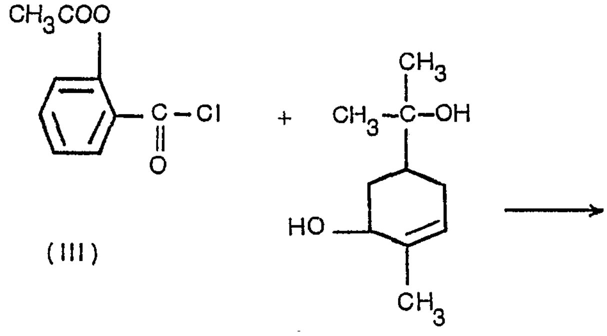

(III) (II) (I)

4. Process according to claim 3, characterized by operating in inert solvents, such as diethylether, tetrahydrofuran or dioxan.

5. Process according to claims 3—4, characterized by operating in the presence of acidity acceptors, such as triethylamine or pyridine.

6. Process according to claims 3—5, characterized by operating at temperatures ranging from about 10 to 100°C, preferably from 20 to 80°C.

**Claims for the Contracting State: AT**

1. Process for the preparation of 2-acetoxybenzoate of 2-methyl-5-(1-hydroxy-1-methyl-ethyl)-cyclohexen-2-yl, having formula (I)

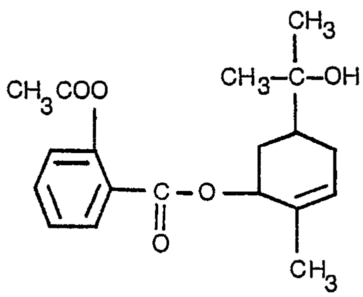

(I)

characterized by reacting acetylsalicylic acid chloride (III) with sobrerol (II), according to the following scheme:

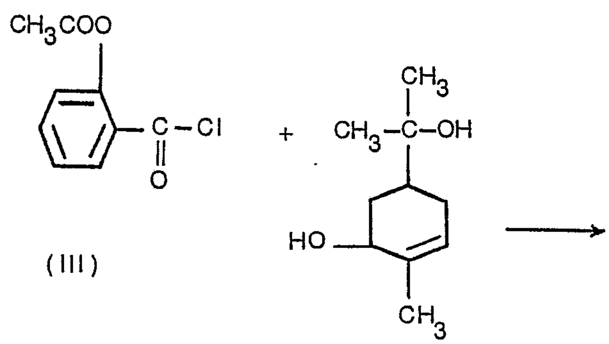

(III) (II) (I).

2. Process according to claim 1, characterized by operating in inert solvents, such as diethylether, tetrahydrofuran or dioxan.

3. Process according to claims 1—2, characterized by operating in the presence of acidity acceptors, such as triethylamine or pyridine.

4. Process according to claims 1—3, characterized by operating at temperatures ranging from about 10 to 100°C, preferably from 20 to 80°C.

**Patentansprüche für die Vertragsstaaten: BE CH DE FR GB IT LI LU NL SE**

1. Als neue Verbindung 2-Methyl-5-(1-hydroxy-1-methyl-ethyl)-cyclohexen-2-yl-2-acetoxybenzoat der Formel (I)

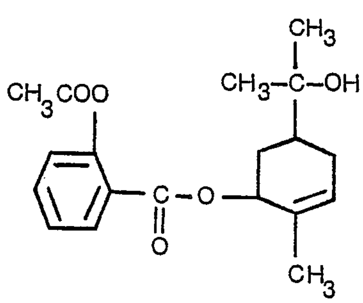

(I)

2. Pharmazeutische Zusammensetzung mit analgetischer, entzündungshemmender, schleimregelnder und Antihusten-Aktivität, dadurch gekennzeichnet, daß sie als aktives Prinzip die Verbindung von Anspruch 1 enthält.

3. Verfahren zur Herstellung von 2-Methyl-5-(1-hydroxy-1-methyl-ethyl)-cyclohexen-2-yl-2-acetoxybenzoat der Formel (I), gekennzeichnet durch Umsetzen von Acetylsalicylsäurechlorid (III) mit Sobrerol (II) nach folgendem Schema:

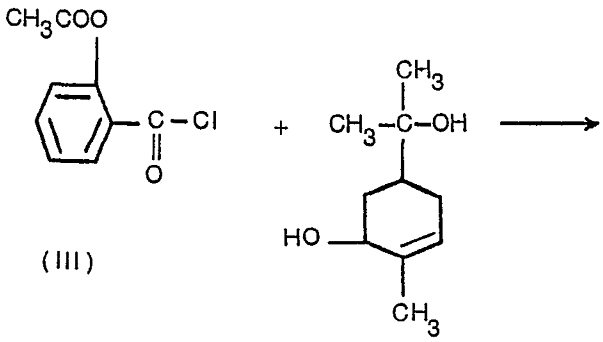

(III)

(II)

(I)

4. Verfahren nach Anspruch 3, gekennzeichnet durch Arbeiten in inerten Lösungsmitteln, wie Diethylether, Tetrahydrofuran oder Dioxan.

5. Verfahren nach den Ansprüchen 3 bis 4, gekennzeichnet durch Arbeiten in Gegenwart von Säureakzeptoren, wie Triethylamin oder Pyridin.

6. Verfahren nach den Ansprüchen 3 bis 5, gekennzeichnet durch Arbeiten bei Temperaturen im Bereich von etwa 10 bis 100°C, bevorzugt von 20 bis 80°C.

**Patentansprüche für den Vertragsstaat: AT**

1. Verfahren zur Herstellung von 2-Methyl-5-(1-hydroxy-1-methyl-ethyl)-cyclohexen-2-yl-2-acetoxybenzoat der Formel (I)

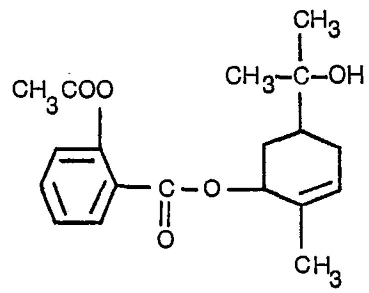

(I)

gekennzeichnet durch Umsetzen von Acetylsalicylsäurechlorid (III) mit Sobrerol (II) nach folgendem Schema:

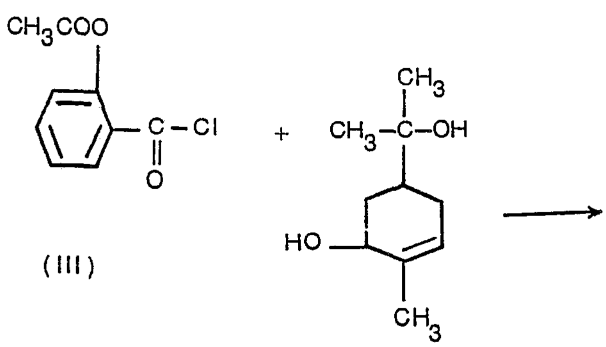

(III)

(II)

(I)

2. Verfahren nach Anspruch 1, gekennzeichnet durch Arbeiten in inerten Lösungsmitteln, wie Diethylether, Tetrahydrofuran oder Dioxan.

3. Verfahren nach den Ansprüchen 1 bis 2, gekennzeichnet durch Arbeiten in Gegenwart von Säureakzeptoren, wie Triethylamin oder Pyridin.

4. Verfahren nach den Ansprüchen 1 bis 3, gekennzeichnet durch Arbeiten bei Temperaturen im Bereich von etwa 10 bis etwa 100°C, bevorzugt von 20 bis 80°C.

**Revendications pour les Etats contractants: BE CH DE FR GB IT LI LU NL SE**

1. En tant que nouveau composé, le 2-acétoxybenzoate de 2-méthyl-5-(1-hydroxy-1-méthyl-éthyl)-cyclohexèn-2-yl, de formule (I)

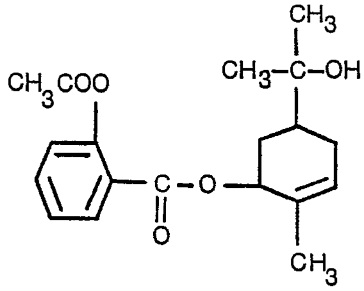

(I)

2. Composition pharmaceutique dotée d'activités analgésique anti-inflammatoire, mucorégulatrice et antitussive, caractérisée en ce qu'elle contient comme principe actif le composé de la revendication 1.

3. Procédé de préparation du 2-acétoxybenzoate de 2-méthyl-5-(1-hydroxy-1-méthyl-éthyl)-cyclohexèn-2-yl, de formule (I), caractérisé par la réaction du chlorure d'acide acétylsalicylique (III) avec du sobrérol (II), selon le schéma suivant:

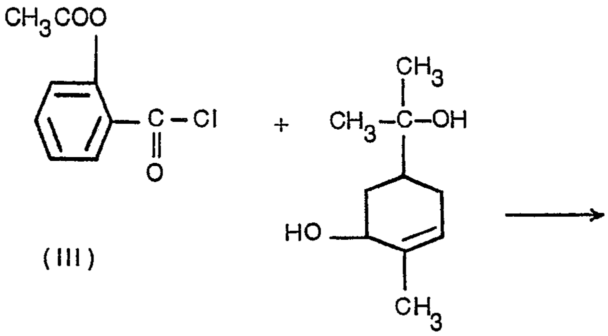

(III) (II) (I)

4. Procédé selon la revendication 3, caractérise en ce qu'on opère dans des solvants inertes tels que le diéthyler, le tétrahydrofuranne ou le dioxanne.

5. Procédé selon les revendications 3 et 4, caractérisé en que qu'on opère en présence d'accepteurs d'acidité tels que la triéthylamine ou la pyridine.

6. Procédé selon les revendications 3 à 5, caractérisé en ce qu'on opère à des températures allant d'environ 10 à 100°C, de préférence de 20 à 80°C.

**Revendications pour l'Etat contractant: AT**

1. Procédé de préparation du 2-acétoxybenzoate de 2-méthyl-5-(1-hydroxy-1-méthyl-éthyl)-cyclohexèn-2-yl, répondant à la formule (I)

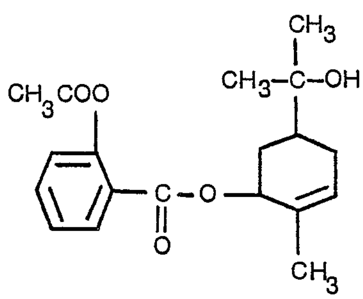

(I)

caractérisé par la réaction du chlorure d'acide acétylsalicylique (III) avec du sobrérol (II), selon le schéma réactionnel suivant:

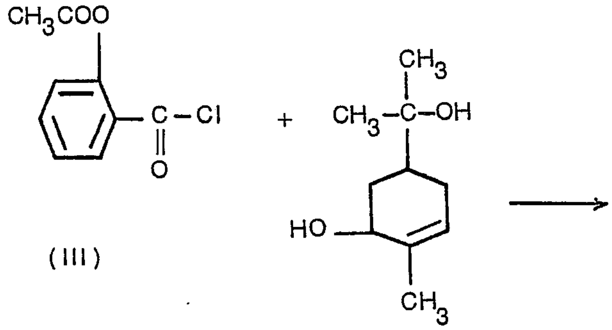

(III) (II) (I)

2. Procédé selon la revendication 1, caractérisé en ce qu'on opère dans des solvants inertes, tels quelle diéthylether, le tétrahydrofuranne ou le dioxanne.

3. Procédé selon les revendications 1 à 2, caractérisé en ce qu'on opère en présence d'accepteurs d'acidité, tels que la triéthylamine ou la pyridine.

4. Procédé selon les revendications 1 à 3, caractérisé en ce qu'on opère à des températures allant d'environ 10 à 100°C, de préférence de 20 à 80°C.